# EUROPEAN PATENT APPLICATION

(11) **EP 4 372 626 A1**
(43) Date of publication of application: **22.05.2024**
(21) Application number: 23195090.8
(22) Date of filing: 04.09.2023
(51) Int. Cl.: G06N 7/01, G06N 7/06, G06Q 10/10, G06Q 50/22, G16H 40/00, G16H 40/20

(54) **INFORMATION PROCESSING PROGRAM, METHOD FOR PROCESSING INFORMATION, AND INFORMATION PROCESSING APPARATUS**

(30) Priority: 16.11.2022 JP 2022183394
(71) Applicant: FUJITSU LIMITED, Kawasaki-shi, Kanagawa 211-8588 (JP)
(72) Inventor: CHANG, Shuang, Kawasaki-shi, Kanagawa, 211-8588 (JP); ASAI, Tatsuya, Kawasaki-shi, Kanagawa, 211-8588 (JP); MARUHASHI, Koji, Kawasaki-shi, Kanagawa, 211-8588 (JP)
(74) Representative: Hoffmann Eitle

(57) **Abstract**

A program for causing a computer to execute processing including: obtaining pieces of combination data each including attribute information indicating attributes of a person and information indicating which choice is selected from choices; generating, for each choice, converted data obtained by converting the information into information indicating whether the choice is selected; identifying, for each choice, based on the converted data, an attribute, from the attributes, that has a correlation greater than a criterion with the selection of the choice and a condition; identifying a common condition among conditions of different choices based on the condition; and determining a plan for improving a selection result of the choice for a target who matches one of the different choices and who matches the common condition based on a result of an analysis between the attributes and the choice by using the converted data matching the common condition.

## Description

### FIELD

The embodiment discussed herein is related to an information processing program, a method for processing information, and an information processing apparatus.

### BACKGROUND

Today, population is aging in various countries. As the population is aging, the number of persons that desire services for cure of elderly persons (hereinafter, referred to as a care service in some cases), which are called a long term care (LTC) service or the like, also increase. To address such a situation, countries, municipalities, and so force streamline the care service provided to elderly persons.

In the related art, there is an attempt to improve a provision plan of the care service (for example, to improve fairness in use of the care service) by using a simulation technique.

Examples of the related art include: Japanese Laid-open Patent Publication No. 2020-046888 is disclosed as related art; and Shuang, C., Wei, Y., and Deguchi, H."Care providers, access to care, and the Long-term Care Nursing Insurance in China: an Agent-based simulation". Social Science & Medicine, vol. 244, pp. 112667, 2019 is also disclosed as related art.

### SUMMARY

### TECHNICAL PROBLEM

According to the above-described related art, in order to improve the provision plan of the care service, the simulation and evaluation of a result of the simulation are repeatedly performed while the conditions of the simulation being changed. Improvement plans obtained through the simulation and the evaluation are plans based on knowledge specific to the field of an evaluator (domain knowledge) and actually improvement plans for whole elderly persons (care receivers) in most cases.

However, in reality, situations that care receivers experience are not uniform, and only improvement plans for whole elderly persons are not necessarily sufficient for improving the provision plan of the care service. In contrast, it is not easy to develop, by trial and error of the evaluator, improvement plans for a target having a specific attribute.

The above-described problems are problems that may arise not only in the provision plan of the care service but also in, for example, review and improvement of aiding measures for a plurality of persons and streamlining of welfare in organizations and groups such as companies and municipalities.

In one aspect, it is an object to aid development of an improvement plan for a target having a specific attribute.

### SOLUTION TO PROBLEM

According to an aspect of the embodiments, there is provided an information processing program including instructions which, when executed by a computer, cause the computer to perform processing including: obtaining a plurality of pieces of combination data, the plurality of pieces of combination data each including attribute information indicative of one or more attributes of a target person and information indicative of which choice is selected by the target person from among a plurality of choices; generating, for each of the plurality of choices, converted data obtained by converting the information into information indicative of whether the choice is selected; identifying, for each of the plurality of choices, based on the converted data, an attribute, out of the one or more attributes, that has a correlation greater than or equal to a predetermined criterion with the selection of the choice and an attribute condition that is a condition of a value of the attribute; identifying an attribute condition common to attribute conditions of two or more different choices based on the attribute condition for each of the plurality of choices; and determining an improvement plan for improving a selection result of the choice for a target person who matches one of the two or more different choices and who matches the common attribute condition based on a result of a causality analysis between the one or more attributes and the choice by using the converted data that matches the common attribute condition.

### ADVANTAGEOUS EFFECTS OF INVENTION

In the one aspect, the development of the improvement plan for the target having the specific attribute may be aided.

### BRIEF DESCRIPTION OF DRAWINGS

FIG. 1 illustrates a functional block diagram of an information processing apparatus;
FIG. 2 illustrates a functional block diagram of a storage unit;
FIG. 3 illustrates an example of data stored in a care receiver data storage unit;
FIG. 4 illustrates a flowchart indicating a series of processes executed by the information processing apparatus;
FIG. 5 illustrates an example of result data;
FIG. 6A illustrates converted data generated by a conversion process;
FIG. 6B illustrates converted data generated by the conversion process;
FIG. 6C illustrates converted data generated by the conversion process; and
FIG. 7 illustrates an example of a hardware configuration of the information processing apparatus.

### DESCRIPTION OF EMBODIMENTS

An embodiment for carrying out the present disclosure will be described with reference to the drawings. The present embodiment is one of forms for realizing the disclosure, and content, an execution order, and the like of each process in the embodiment may be changed as appropriate within a range in which the disclosure may be realized.

### [Functional Block]

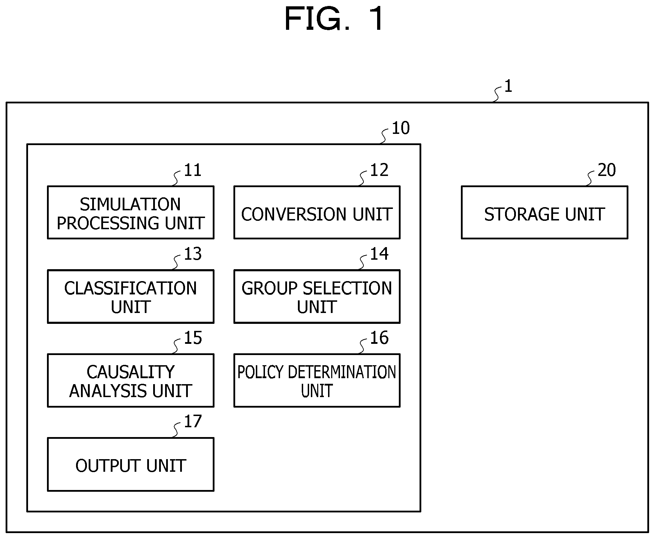

FIG. 1 is a functional block diagram of an information processing apparatus 1 according to the present embodiment. For example, the information processing apparatus 1 is an information processing apparatus such as a personal computer (PC) or a server apparatus. As illustrated in FIG. 1, the information processing apparatus 1 includes a processing unit 10 and a storage unit 20 as functional units.

The processing unit 10 includes a simulation processing unit 11, a conversion unit 12, a classification unit 13, a group selection unit 14, a causality analysis unit 15, a policy determination unit 16, and an output unit 17. The simulation processing unit 11 executes a simulation process, which will be described later. The conversion unit 12, the classification unit 13, the group selection unit 14, the causality analysis unit 15, and the policy determination unit 16 execute a policy determination process, which will be described later. The output unit 17 outputs various types of data. The details of processes executed by the functional units, the details of the simulation process, and the details of the policy determination process will be described later.

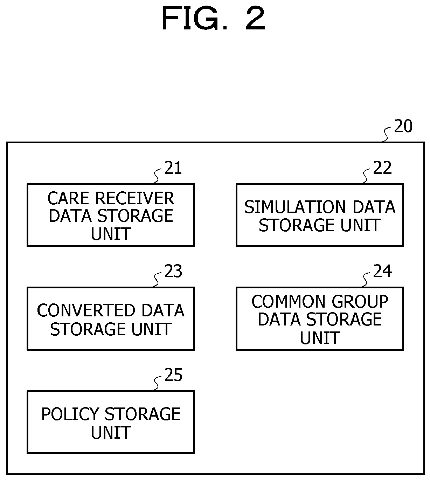

FIG. 2 is a functional block diagram of the storage unit 20. As illustrated in FIG. 2, the storage unit 20 includes a care receiver data storage unit 21, a simulation data storage unit 22, a converted data storage unit 23, a common group data storage unit 24, and a policy storage unit 25.

The care receiver data storage unit 21 stores data to be used in a simulation executed by the simulation processing unit 11. The simulation data storage unit 22 stores various types of setting information, simulation results, and the like used in the simulation executed by the simulation processing unit 11. The converted data storage unit 23, the common group data storage unit 24, and the policy storage unit 25 store various types of data generated and used in the policy determination process, which will be described later.

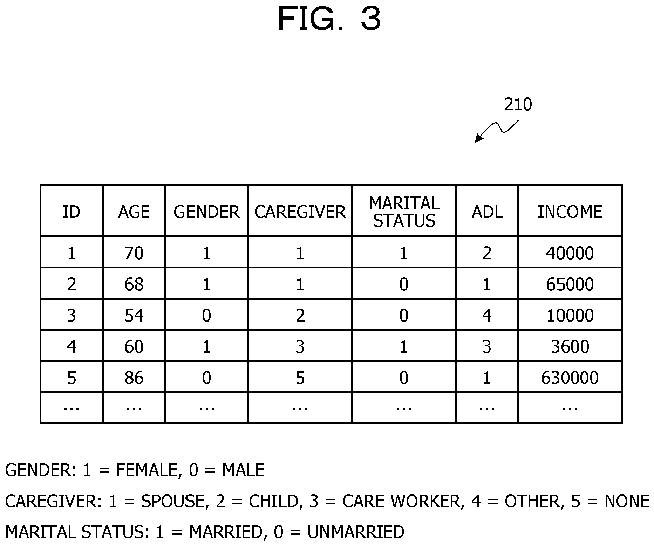

FIG. 3 is a diagram illustrating an example of the data stored in the care receiver data storage unit 21. Care receiver data 210 is data indicating attributes of the care receiver for each of a plurality of the care receivers to be simulated. The care receiver data 210 illustrated in FIG. 3 is, for example, data in a table format including data items of an identifier (ID), an age, a gender, a caregiver, a marital status, activities of daily living (ADL), and an income as the data items.

In the care receiver data 210, the ID is identification information that may uniquely identify each of the care receivers. The age is information indicating the age of the care receiver. The gender is information indicating the gender of the care receiver. The caregiver is information indicating whether a person who cares for the care receiver is present and who cares for the care receiver. The marital status is information indicating whether the care receiver is in a married state or an unmarried state.

The ADL are information indicating an evaluation of the ADL of the care receiver. The ADL refer to basic activities desired for the care receiver to live a daily life. The ADL as a data item in FIG. 3 are, for example, a data item in which the evaluation of the ADL of the care receiver is indicated by a value that increases as the difficulty of the activities increases. The income is information indicating the household income of a household to which the care receiver belongs.

The data items included in the care receiver data 210 of FIG. 3 are exemplary. The data items may be increased or decreased as appropriate. For example, in addition to the data items illustrated in FIG. 3, information such as an insurance subscription status of the care receiver, a health condition of the care receiver, the place of residence of the care receiver, and whether the care receiver lives with an other person or alone may be set as the data items. The care receiver data 210 may be data indicating the attributes of a plurality of real care receivers or virtual data generated based on statistical information such as an actual population ratio or investigation results of senior citizens.

[Description of Processes Executed by Information Processing Apparatus 1]

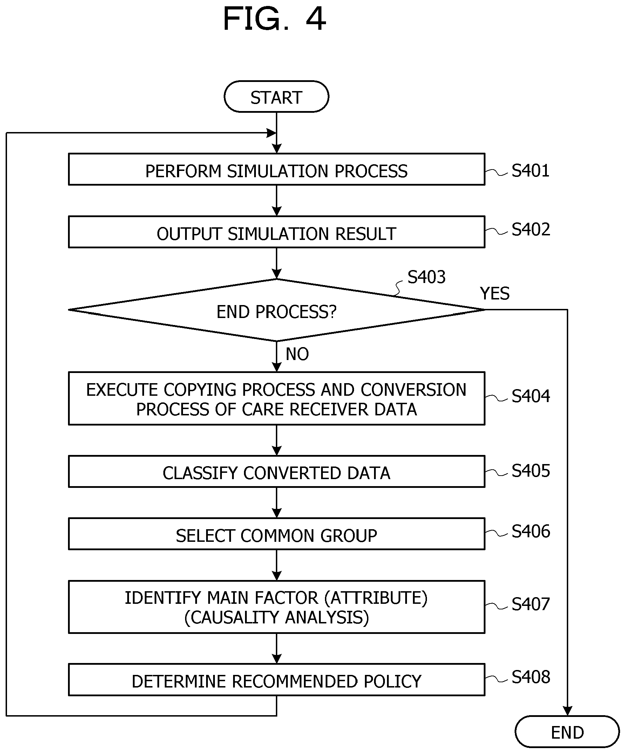

FIG. 4 is a flowchart illustrating a series of processes executed by the information processing apparatus 1 according to the present embodiment. Referring to FIG. 4, the simulation processing unit 11 executes the simulation process (S401). According to the present embodiment, a simulation technique using agent-based modeling (ABM) disclosed in the following article is used: "Shuang, C., Wei, Y., and Deguchi, H."Care providers, access to care, and the Long-term Care Nursing Insurance in China: an Agent-based simulation". Social Science & Medicine, vol. 244, pp. 112667, 2019"

As a result of the simulation process, data in which each data row of the care receiver data 210 is associated with information indicating a corresponding selection result of a care service is generated. Hereinafter, this data in which each data row of the care receiver data 210 is associated with the information indicating the corresponding selection result of the care service generated as the result of the simulation process may be referred to as "result data". The simulation processing unit 11 stores the generated result data to the simulation data storage unit 22.

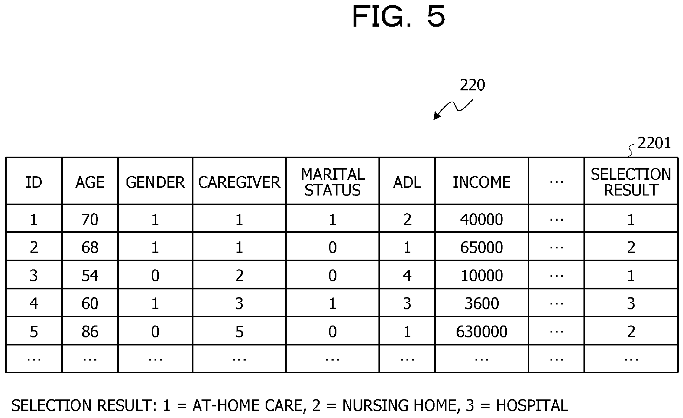

FIG. 5 is a diagram illustrating an example of the result data. Result data 220 of FIG. 5 is data in a table format in which the data item of a selection result 2201 is associated with the above-described care receiver data 210. The selection result 2201 is information indicating, for each care receiver, the selection result of the care service, determined by the simulation process.

Returning to the description with reference to FIG. 4, the simulation processing unit 11 outputs a result of the simulation executed in step S401 via the output unit 17 (S402). For example, the simulation processing unit 11 displays the results of the simulation on a display screen of a display device 78, which will be described later. As the result of the simulation, for example, an index for evaluating fairness of use of the care service such as an age-by-age or a household income-by-household income use ratio of the care service is output. Examples of the index for evaluating the fairness of the use of the care service include, for example, data obtained by outputting, in a graphical representation, the relationship between an attribute on which attention is focused (for example, income) and the cumulative number of times of use of the care service for a specific care service. A frequency of use or a rate of use of the care service may be set as the item to be output instead of the cumulative number of times of use. Examples of the index for evaluating the fairness also include data indicating a nonuse rate of the care service (the ratio of care receivers who use none of the care services) in accordance with the difference in income. The income in the description herein is an example of the attribute on which the attention is focused in the evaluation of the fairness of use of the care service and may be replaced with an other attribute such as, for example, a resident area of the care receiver.

Next, the simulation processing unit 11 determines whether to end the series of processes illustrated in FIG. 4 (S403). As a method for determining whether to end, various forms are conceivable. For example, the information processing apparatus 1 accepts the result of determining whether the result of the simulation output in step S402 is good from a user who is an output destination of the result of the simulation. In a case where the accepted determination result indicates that the result of the simulation is good (S403, YES), the simulation processing unit 11 ends the series of processes illustrated in FIG. 4. In contrast, in a case where the accepted determination result indicates that the result of the simulation is not good (S403, NO), the policy determination process described step by step below from steps S404 to S408 is executed.

In step S404, the conversion unit 12 executes a copying process and a conversion process of the result data 220 (S404). As a result of the processes of step S404, converted data obtained by converting the result data 220 is generated for each selection result of the care service.

After that, the classification unit 13 executes a classification process to classify the converted data for individual pieces of the converted data generated for the respective selection results of the care service (S405). Based on a result of the classification process, the group selection unit 14 executes a process of selecting a common group (S406). The causality analysis unit 15 executes a causality analysis of the data of the common group, thereby identifying a data item (attribute) that is a main factor for selection for each of the care services (S407). The policy determination unit 16 determines a recommended policy based on the main factor identified in step S407 (S408). After the process in step S408, the simulation process described in step S401 is executed again by using the determined recommended policy, and the processes of steps S402 and S403 are also executed again.

The details of the process of each of S404 to S408 and the data and terms related to the processes will be described later.

### [Details of Copying Process and Conversion Process (Step S404) of Result Data 220]

The details of the processes of step S404 are described. First, the conversion unit 12 identifies the number of types of data values of the selection result 2201 which is a data item included in the result data 220. For example, in the case of the result data 220 illustrated in FIG. 5, three types (1, 2, and 3) of the data values exist. Thus, the conversion unit 12 makes three copies of the result data 220. Here, it may be said that the number of types of the data values of the selection result 2201 is the number of types of care services selected in the simulation process.

Next, the conversion unit 12 performs the conversion process on each piece of the copied result data 220. The conversion process is a process of changing the data values of the selection result 2201. For example, the conversion unit 12 converts each piece of the copied result data 220 such that the data value of the selection result 2201 is binary data indicating whether a corresponding care service is selected.

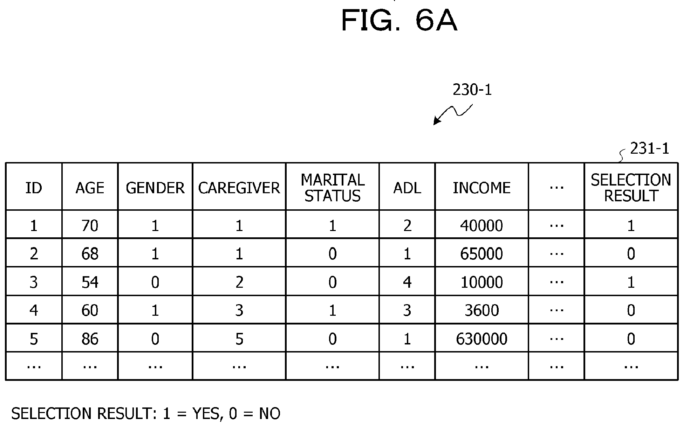

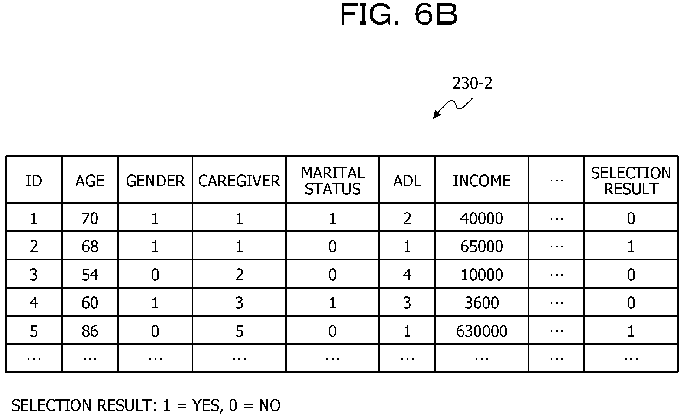

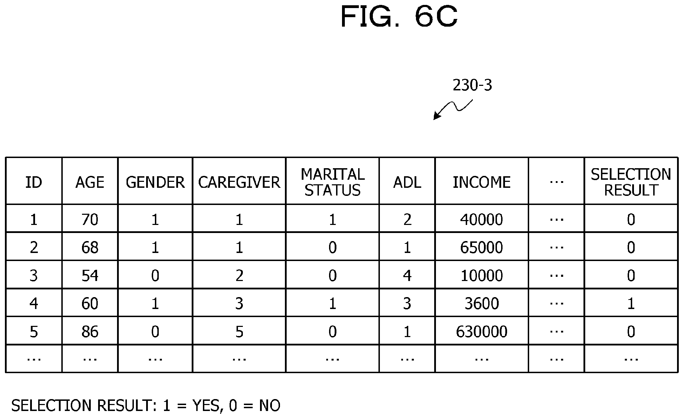

FIGs. 6A, 6B, and 6C are diagrams illustrating converted data 230-1, 230-2, and 230-3 generated by the conversion process. The converted data 230-1 is converted data in a case where the care service is at-home care. The converted data 230-2 is converted data in a case where the care service is a nursing home. The converted data 230-3 is converted data in a case where the care service is a hospital. For example, in the case of the converted data 230-1, unlike the result data 220, the selection result 231-1 is a data item of binary data indicating whether the at-home care is selected as the care service. For example, the data is converted such that the data value is 1 in a case where the at-home care is selected as the care service (YES) and the data value is 0 in a case where a service other than the at-home care is selected as the care service (NO). Likewise, the data is converted so as to indicate whether the nursing home is selected as the care service in the case of the converted data 230-2 and whether the hospital is selected as the care service in the case of the converted data 230-3. The conversion unit 12 stores converted data 230-1, 230-2, and 230-3 generated by the conversion process in the converted data storage unit 23.

### [Details of Classification Process of Result Data 220 (Step S405)]

The details of the process in step S405 are described. The classification unit 13 executes the classification process of each of the converted data 230-1, the converted data 230-2, and the converted data 230-3 of the corresponding type of the care service stored in the converted data storage unit 23.

The classification unit 13 reads the converted data of the type of any one of the care services out of the data stored in the converted data storage unit 23. As an example, it is assumed that the classification unit 13 reads the converted data 230-1 from the converted data storage unit 23. When the classification unit 13 executes the classification process of the read converted data 230-1, the classification unit 13 identifies attribute values highly correlated to the selection of the care service (at-home care in the case of the converted data 230-1). The attribute values are the conditions of the data values of data items or combinations thereof. For the classification process, for example, the technique disclosed in Japanese Laid-open Patent Publication No. 2020-046888 may be adopted. Alternatively, an other technique may be adopted.

In the present embodiment, it is assumed that the attribute values "the gender is female (data value = 1)", "the ADL is smaller than 4", and "the household income is higher than 2239" are identified through the classification process of the converted data 230-1. For example, based on the result of the classification process, there is a high tendency that the care receivers who select the at-home care as the care service have attribute values of "the gender is female (data value = 1)", "the ADL is smaller than 4", and "the household income is higher than 2239". In the following description, an attribute value highly correlated to the selection of a care service for each type of the care services may be referred to as an "attribute condition". For example, the attribute conditions obtained from the converted data 230-1 may be described as "attribute conditions of at-home care".

The classification unit 13 also identifies the attribute values highly correlated to the selection of the care services in a similar manner for the converted data 230-2 and the converted data 230-3. After the classification unit 13 has executed the classification process of all the converted data stored in the converted data storage unit 23, the classification unit 13 ends the process in step S405.

### [Details of Common Group Selection (Step S406)]

The details of the process in step S406 are described. Based on the result of the classification process in step S405, the group selection unit 14 executes the process of selecting (identifying) the common group. Herein, the common group refers to a set of care receivers corresponding to an attribute condition common among the attribute conditions of two or more different care services.

The description is made with a specific example. First, as described above, it is assumed that the attribute conditions of "the gender is female (data value = 1)", "the ADL are smaller than 4", and "the income is higher than 2239" are identified as the attribute conditions of the at-home care. As an example for the description, it is assumed that the attribute conditions of "the gender is female (data value = 1)", "the ADL are smaller than 4", and "no caregiver is present (data value = 5)" are identified as the attribute conditions of the nursing home. In this case, the attribute conditions common to the "attribute conditions of the at-home care" and the "attribute conditions of the nursing home" are "the gender is female (data value = 1)" and "the ADL are smaller than 4". The common group in this case means a set of care receivers corresponding to the conditions of "the gender is female (data value = 1)" and "the ADL are smaller than 4".

For example, as in the above example, the group selection unit 14 identifies the attribute conditions common between the attribute conditions of two different care services for each of combinations of care services. Based on the identified attribute conditions, the group selection unit 14 selects a common group for each of the combinations of care services. The group selection unit 14 stores information identifying the common group for each of the combinations of care services to a common group data storage unit 24. Alternatively, the group selection unit 14 may extract the data corresponding to the common groups from the converted data storage unit 23 and store the extracted data to the common group data storage unit 24.

### [Identification of Data Item (attribute) Being Main Factor (S407)]

The details of the process in step S407 are described. Based on each of the common groups selected by the process in step S406, the causality analysis unit 15 identifies a data item (attribute) that influences the selection of each of the care services. Hereinafter, the data item (attribute) that influences the selection of each care service may be referred to as a "main factor".

For example, for each of the common groups identified through the process in step S406, the causality analysis unit 15 executes the causality analysis between the data items by using the converted data corresponding to the common group. As a result of the causality analysis, the causality analysis unit 15 identifies as the main factor the data item determined to have causal relationship with the selection of each care service.

For the causality analysis, for example, algorithms such as a Peter and Clark (PC) algorithm, a greedy fast causal inference (GFCI), and a linear non-Gaussian acyclic model (DirectLiNGAM) may be used. A single algorithm may be used for the causality analysis, or a plurality of causality analysis algorithms may be used in combination. In a case where a plurality of causality analysis algorithms are used in combination, for example, the causality analysis unit 15 may identify, as the main factor, a data item determined to be a main factor in a greater than or equal to a predetermined number of the plurality of algorithms in combination or all of the plurality of algorithms used in combination.

For example, the common group which has been described in the above example and in which the common attribute conditions are "the gender is female (data value = 1)" and "the ADL are smaller than 4" is described again as the example. It is assumed that, as a result of the causality analysis, the age and the income are determined to have the causal relationships with the selection of the at-home care as the care service. In this case, the causality analysis unit 15 identifies that the age and the annual income are the main factors for the selection of the at-home care in the common group in which the attribute conditions are "the gender is female (data value = 1)" and "the ADL are smaller than 4".

As described above, the causality analysis unit 15 identifies the main factor for the selection of each care service of each common group.

### [Determination of Recommended Policy (S408)]

The details of the process in step S408 are described. The policy determination unit 16 determines the recommended policy based on the result of the process in step S407. The policy determination unit 16 determines a recommended policy based on the attribute condition for the common group and the main factor of selection for each care service. The recommended policy is an improvement plan for improving provision of the care service.

For example, as in the above-described example, a case is considered in which the age and the annual income are identified as the main factors for the selection of the at-home care in the common group in which the attribute conditions are "the gender is female (data value = 1)" and "the ADL are smaller than 4". In this case, for example, the policy determination unit 16 determines, as the recommended policy related to the at-home care, that a cash benefit is given to women of the ADL of smaller than 4. Alternatively, in consideration of the age and the annual income being the main factors, for example, a recommended policy of giving a case benefit to women of the ADL of smaller than 4 and at an age older than or equal to a predetermined age may be determined.

The policy determined here is different from the policy for the care receivers, and the policy for a specific group in accordance with the attribute conditions and for a specific care service is determined. As described above, the policy determination unit 16 determines the recommended policy based on the main factor for each common group. The policy determination unit 16 stores the determined recommended policy to the policy storage unit 25.

### [Re-execution of Simulation Process]

After the process in step S408, the simulation processing unit 11 executes the simulation process (S401) again by using the determined recommended policy. For example, the simulation processing unit 11 generates data in which the recommended policy is applied to the care receiver data 210 to execute the simulation process by using the generated data. As a specific example, in a case where the recommended policy is a policy in which the cash benefit is given to women of the ADL of smaller than 4, the simulation processing unit 11 identifies data rows satisfying the conditions of "the gender is female (data value = 1)" and "the ADL are smaller than 4" among the data included in the care receiver data 210. The simulation processing unit 11 adds a predetermined value (a value corresponding to an assumed benefit amount) to the data value of the annual income of each of the identified data rows to generates data to which the recommended policy has been applied. The simulation processing unit 11 executes the simulation process by using the data to which the recommended policy has been applied (S401) and outputs a result of the simulation (S402). The simulation processing unit 11 performs the determination in the step S403. Depending on the determination result, re-execution of the processes in and after step S404 or ending of the series of processes illustrated in FIG. 4 is selected.

### [Effects and Significance of Embodiment]

According to the present embodiment, the classification process of each type of the care services and the selection of the common group are executed before causality analysis for policy determination. First, for each type of the care services, the attribute values (attribute conditions) highly correlated to the selection of the care service are identified. Thus, data highly correlated to the selection of the care service is selected as data to be used for the causality analysis to be executed later. In addition, the recommended policy is not for the care receivers as a whole. A policy more specialized for the care receivers of specific attributes is generated.

In addition, the data belonging to the above-described common group is used for the causality analysis. Thus, in the causality analysis, the influence of the causal relationship that does not depend on the types of the care services (for example, a usual causal relationship common to the whole care receivers) is suppressed. Accordingly, the causal relationship between each care service and the attribute obtained by the causality analysis is more specialized in the care service.

The common group has the attributes highly correlated to the selection of a care service in a plurality of care services. When the common group is set as a target of the causality analysis, a policy specialized for the care receivers having a more important attribute in the selection of the care service is generated.

From the above description, it is expected that the recommended policy determined by the processes of the information processing apparatus 1 is a policy specialized for a specific care service or care receivers having a specific attribute.

In the case described as the related art, the simulation and the evaluation of the result of the simulation are repeatedly performed while the conditions of the simulation being changed. According to the related art, the improvement plan is obtained by trial and error of an evaluator, and a large number of times of the simulation, for example, a large cost (funds, computer resources, and time) is desired. For this reason, an increase in efficiency of the improvement (suppression of the cost) of the provision plan of the care service is desired. With the processes described in the present embodiment, the information processing apparatus 1 determines the policy for the specific group based on the simulation result. Accordingly, it is expected that a useful policy (improvement plan) may be obtained with a smaller number of times of trials. Since the evaluation may be performed by re-executing the simulation in which the obtained policy is reflected, the provision plan of the care service may be improved at a lower cost compared to the related art.

### [Example of Hardware Configuration]

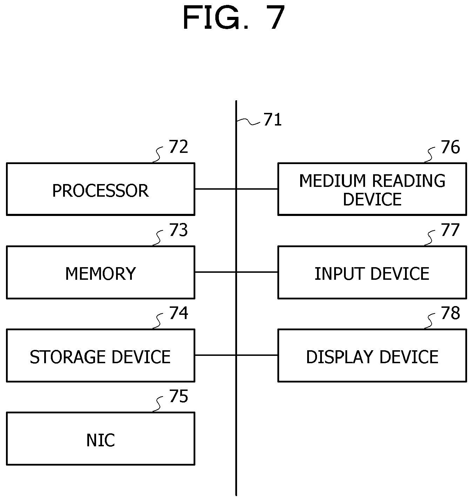

FIG. 7 is a diagram illustrating an example of a hardware configuration of the information processing apparatus 1 according to the present embodiment. The information processing apparatus 1 is an information processing apparatus that includes, for example, a processor 72, a memory 73, a storage device 74, a network interface controller (NIC) 75, a medium reading device 76, an input device 77 and the display device 78. These devices and the like are coupled to each other via a bus 71.

The processor 72 performs various types of operation control in the information processing apparatus 1. The memory 73 and the storage device 74 store a program that executes various processes described in the present embodiment and various types of data used for the various processes. The processor 72 is a generic name of hardware circuits such as a central processing unit (CPU), a microprocessor unit (MPU) and an application-specific integrated circuit (ASIC). The storage device 74 is a storage medium such as, for example, a hard disk drive (HDD) or a solid-state drive (SSD).

The functional units included in the processing unit 10 illustrated in FIG. 1 may be realized when the processor 72 reads the program stored in the memory 73 or the storage device 74 and executes the processes and control. Each of the memory 73 and the storage device 74 may function as the storage unit 20 illustrated in FIGs. 1 and 2. According to the present embodiment, instead of the storage device 74, an external storage device different from the information processing apparatus 1 may function as the storage unit 20.

The NIC 75 is hardware to be used for data transmission/reception to/from other devices via a wired or wireless network.

The medium reading device 76 is a device for reading data from a recording medium and includes, for example, a disk drive that reads data stored in a disk medium such as a compact disc read-only memory (CD-ROM) or a digital versatile disk ROM (DVD-ROM), a card slot that reads data stored in a memory card, or the like. A part or all of the data stored in the above-described storage unit 20 may be stored in the recording medium readable by using the medium reading device 76.

The input device 77 is a device that accepts input and designation from the user of the information processing apparatus 1. The display device 78 displays various types of information under the control of the processor 72. Examples of the input device 77 include, for example, a keyboard, a mouse, and a touch pad. The display device 78 is, for example, a liquid crystal display. According to the present embodiment, for example, a touch panel having the functions of the input device 77 and the display device 78 may be used.

Although it has been described that the information processing apparatus 1 which is a single apparatus executes each of the processes according to the present embodiment, the processes executed by the functional units of the information processing apparatus 1 may be distributed to and executed by a plurality of information processing apparatuses. A form may also be employed in which the information processing apparatus 1 is a server apparatus, and the output of the simulation result and the input of the determination on the quality of the result by the user are performed by a client apparatus able to communicate with the information processing apparatus 1 which is the server apparatus.

As a specific example, the application in developing the provision plan of the care service is exemplified according to the present embodiment. However, the content described according to the present embodiment is not only aimed at the provision plan of the care service. The technique described according to the present embodiment may also be applied to other applications such as, for example, review and improvement of aiding measures for a plurality of persons and streamlining of welfare in organizations and groups such as companies and municipalities.

## Claims

1. An information processing program comprising instructions which, when executed by a computer, cause the computer to perform processing comprising:
obtaining a plurality of pieces of combination data, the plurality of pieces of combination data each including attribute information indicative of one or more attributes of a target person and information indicative of which choice is selected by the target person from among a plurality of choices;
generating, for each of the plurality of choices, converted data obtained by converting the information into information indicative of whether the choice is selected;
identifying, for each of the plurality of choices, based on the converted data, an attribute, out of the one or more attributes, that has a correlation greater than or equal to a predetermined criterion with the selection of the choice and an attribute condition that is a condition of a value of the attribute;
identifying an attribute condition common to attribute conditions of two or more different choices based on the attribute condition for each of the plurality of choices; and
determining an improvement plan for improving a selection result of the choice for a target person who matches one of the two or more different choices and who matches the common attribute condition based on a result of a causality analysis between the one or more attributes and the choice by using the converted data that matches the common attribute condition.

2. The information processing program according to claim 1, wherein
the identifying of the attribute condition executes a classification of the converted data, thereby identifying the attribute, out of the one or more attributes, that has the correlation greater than or equal to the predetermined criterion with the selection of the choice and the attribute condition that is the condition of the value of the attribute.

3. The information processing program according to claim 1, wherein
the plurality of pieces of combination data are generated by a simulation based on the information indicative of the one or more attributes for each of a plurality of the target persons, and wherein
the process further includes
outputting a result of the simulation based on the information indicative of the one or more attributes for each of the plurality of target persons for whom a data value has been changed based on the improvement plan, and
performing, in a case where a quality determination on the result of the simulation is received and a determination result of the received quality determination indicates that the result of the simulation is not good, based on the plurality of pieces of combination data generated by the simulation which corresponds to the determination result, the generating of the converted data, the identifying of the attribute condition, the identifying of the common attribute condition, and the generating of the improvement plan.

4. The information processing program according to claim 1, wherein
the determining includes
identifying an attribute, from among the one or more attributes, that has a causal relationship with a specific choice included in the plurality of choices based on the result of the causality analysis, and
determining an improvement plan that improves the identified attribute that has the causal relationship.

5. The information processing program according to claim 4, wherein
the causality analysis is executed by using a plurality of causality analysis algorithms, and
the attribute that is determined to have the causal relationship with the specific choice in a causality analysis in which two or more causality analysis algorithms a number of which has been predetermined out of the plurality of causality analysis algorithms are used is identified as the attribute that has the causal relationship with the specific choice.

6. An information processing method implemented by a computer, the information processing method comprising:
obtaining a plurality of pieces of combination data, the plurality of pieces of combination data each including attribute information indicative of one or more attributes of a target person and information indicative of which choice is selected by the target person from among a plurality of choices;
generating, for each of the plurality of choices, converted data obtained by converting the information into information indicative of whether the choice is selected;
identifying, for each of the plurality of choices, based on the converted data, an attribute, out of the one or more attributes, that has a correlation greater than or equal to a predetermined criterion with the selection of the choice and an attribute condition that is a condition of a value of the attribute;
identifying an attribute condition common to attribute conditions of two or more different choices based on the attribute condition for each of the plurality of choices; and
determining an improvement plan for improving a selection result of the choice for a target person who matches one of the two or more different choices and who matches the common attribute condition based on a result of a causality analysis between the one or more attributes and the choice by using the converted data that matches the common attribute condition.

7. An information processing apparatus comprising:
a memory; and
a processor circuit coupled to the memory, the processor circuit being configured to perform processing including:
obtaining a plurality of pieces of combination data, the plurality of pieces of combination data each including attribute information indicative of one or more attributes of a target person and information indicative of which choice is selected by the target person from among a plurality of choices;
generating, for each of the plurality of choices, converted data obtained by converting the information into information indicative of whether the choice is selected;
identifying, for each of the plurality of choices, based on the converted data, an attribute, out of the one or more attributes, that has a correlation greater than or equal to a predetermined criterion with the selection of the choice and an attribute condition that is a condition of a value of the attribute;
identifying an attribute condition common to attribute conditions of two or more different choices based on the attribute condition for each of the plurality of choices; and
determining an improvement plan for improving a selection result of the choice for a target person who matches one of the two or more different choices and who matches the common attribute condition based on a result of a causality analysis between the one or more attributes and the choice by using the converted data that matches the common attribute condition.
